# EUROPÄISCHE PATENTSCHRIFT

(11) **EP 2 365 868 B1**
(45) Veröffentlichungstag und Bekanntmachung des Hinweises auf die Patenterteilung: **24.04.2019**
(21) Anmeldenummer: 09756425.6
(22) Anmeldetag: 06.11.2009
(51) Int. Cl.: B01D 61/42

(54) **ELEKTRO-ULTRAFILTRATIONSVERFAHREN ZUR BESTIMMUNG DER LÖSLICHKEIT UND MOBILITÄT VON SCHWERMETALLEN UND/ODER SCHADSTOFFEN FÜR ABFALL- UND ALTLASTENMATERIALEN SOWIE SCHWERMETALL- UND/ODER SCHADSTOFF KONTAMINIERTE BÖDEN**
ELECTROULTRAFILTRATION METHOD OF DETERMINING THE SOLUBILITY AND MOBILITY OF HEAVY METALS AND/OR POLLUTANTS FOR WASTE MATERIALS AND OLD CONTAMINATED MATERIALS AND ALSO SOILS CONTAMINATED WITH HEAVY METAL AND/OR POLLUTANT
PROCÉDÉ D ÉLECTRO-ULTRAFILTRATION POUR LA DÉTERMINATION DE LA SOLUBILITÉ ET DE LA MOBILITÉ DE MÉTAUX LOURDS ET/OU DE POLLUANTS POUR DES MATÉRIAUX DE DÉCHETS ET DE RÉSIDUS TOXIQUES, AINSI QUE POUR DES SOLS CONTAMINÉS PAR DES MÉTAUX LOURDS ET/OU DES POLLUANTS

(30) Priorität: 12.11.2008 DE 102008056884
(43) Veröffentlichungstag der Anmeldung: 21.09.2011
(73) Patentinhaber: Südzucker AG, 68165 Mannheim (DE)
(72) Erfinder: HORN, Dietmar, 97246 Eibelstadt (DE)
(74) Vertreter: Schrell, Andreas
(86) Internationale Anmeldenummer: PCT/EP2009/007954
(87) Internationale Veröffentlichungsnummer: WO 2010/054778

(56) Entgegenhaltungen:
- DE-A1- 2 103 111
- DE-A1- 19 500 376
- US-A- 3 784 460
- US-A- 4 331 525
- Dietmar Horn: "Bestimmung von Mikronährstoffen und Schwermetallen in Böden mit dem Verfahren der Elektro-Ultrafiltration (EUF) durch Zugabe von DTPADetermination of micronutrients and heavy metals in soils using electro-ultrafiltration (EUF) technique by addition of DTPA", JOURNAL OF PLANT NUTRITION AND SOIL SCIENCE, vol. 169, no. 1, 1 February 2006 (2006-02-01), pages 83-86, XP055261203, ISSN: 1436-8730, DOI: 10.1002/jpln.200520549

## Beschreibung

Die vorliegende Erfindung betrifft ein Verfahren zur Bestimmung der unterschiedlichen Löslichkeit und Mobilität von Schwermetallen und/oder Schadstoffen in Proben, insbesondere in Abfall- und Altlastenmaterialien, sowie in Schwermetall und/oder Schadstoff kontaminierten Böden, welches sich durch eine besonders schnelle, kostengünstige und verlässliche Verfahrensweise auszeichnet.

Abfall- und Altlastenmaterialien wie Bauschutt, Bleihüttengranulate, Gießereirestsande, Pochsand, oder Asche von Müllverbrennungsvorgängen sowie Schwermetall und/oder Schadstoff kontaminierte Böden fallen in industrialisierten, aber auch Übergangsgesellschaften in zunehmendem Umfang an. Sie stellen für die Umwelt und die Menschheit ein beachtliches Problem dar, nicht zuletzt aufgrund der in aller Regel in ihnen vorhandenen Schwermetalle und/oder Schadstoffe. Selbige erweisen sich häufig als toxisch oder aus anderen Gründen als unerwünscht. Entsprechend ihres Gefährdungspotentials müssen die Materialien entweder in geeigneten Deponien abgelagert, weiterverarbeitet oder sonst wie verbracht werden.

Ein bekanntes Verfahren sieht vor, den Austrag dieser Elemente aus diesen Materialien mittels so genannter Lysimeter zu simulieren, um das Gefährdungspotenzial abzuschätzen, was jedoch Untersuchungszeiten von mehreren Jahren mit sich bringt. Mittels der Säulenmethode lassen sich mit Ergebnissen der Lysimeter vergleichbare Resultate in verkürzter, in der Regel etwa 4 bis 6 Wochen dauernder Weise ermitteln. Aufgrund dieser langfristigen Extraktionszeiten sind derartige Methoden jedoch für Routineuntersuchungen nachteilig.

Aus der US 3,784,460 ist es bekannt, dass Ionen und Schadstoffe im Rahmen von Elektrodepositionsverfahren mittels kombinierter Elektrodialyse und Ultrafiltration kontrolliert werden können.

Zur Bestimmung von pflanzlich genutzten Nährstoffen in Böden wird in zunehmendem Maße das Verfahren der Elektro-Ultrafiltration (EUF) eingesetzt. Im Rahmen dieses Verfahrens werden zu analysierende Bodenproben in Wasser suspendiert und einem elektrischen Feld ausgesetzt. Die zur Erzeugung des elektrischen Feldes eingesetzten Elektroden sind durch Ultrafiltrationsmembranen von der Suspension getrennt, sodass beim Anlegen der elektrischen Spannung die sich in der Suspension befindlichen elektrisch geladenen Nährstoffe, beispielsweise Nitrat, Phosphat, Sulfat oder Natrium, Calcium, Magnesium, Kalium, die als negativ oder positiv geladene Ionen vorliegen, durch die Ultrafiltrationsmembranen hinweg zu der jeweils entgegengesetzt geladenen Elektrode wandern und dort zusammen mit den jeweiligen elektrolytischen Spaltprodukten des Wassers in einem wässrigen Filtrat aufgefangen werden, in welchem sie später analysiert werden (VDLUFA-Methodenbuch I, 1997, 2002, VDLUFA-Verlag, Darmstadt). In DE 2 103 111 wird ein Elektro-Ultrafiltergerät zur Bodenuntersuchung offenbart, das mit einem Potentiometer verbunden ist, welches die Betriebsspannung zum Entziehen der Nährionen nach jedem der sich aneinanderreihenden bestimmten Zeitabschnitte sprunghaft erhöht.

Die DE 195 00 376 A1 offenbart ein Elektro-Ultrafiltrationsverfahren, wonach die Extraktion bei 400 V Spannung und bei Temperaturen von 80 °C in den meisten Fraktionen durchgeführt und in 15 Minuten - noch bevor die Bodenkolloide die Ultrafilter zusetzen können - beendet wird.

Im Rahmen der EUF-basierten Bestimmung von wasserlöslichen Nährstoffen in Böden werden gelegentlich auch bestimmte Mikronährstoffe nachgewiesen. Dabei werden die mittels Wasser löslichen Nährstoffe zunächst in herkömmlicher Weise unter Einsatz von voll entsalztem Wasser in einer ersten und zweiten Fraktion extrahiert. Im Anschluss der zweiten Fraktion wird während des Verfahrens ein Komplexierungsmittel hinzu gegeben, um bestimmte Mikronährstoffe und Schwermetalle zu extrahieren (J. Plant Nutr. Soil Sci., 2006, 169, 83-86). Die derart extrahierten Mikronährstoffe werden in einer einzigen Fraktion erfasst. Sie stehen in Beziehung zu den für die Pflanzen verfügbaren Mengen, wie in landwirtschaftlichen Feldversuchen ermittelt wurde. Für darüber hinausgehende Fragestellungen zur Löslichkeit von Schwermetallen und/oder Schadstoffen sowie der Mobilität und Rate der Freisetzung gibt eine einzige Fraktion nur begrenzte Aussagekraft.

Der vorliegenden Erfindung liegt daher das technische Problem zugrunde, ein Bestimmungsverfahren für Schwermetallionen und/oder Schadstoffe in Proben, insbesondere Abfall- und Altlastenmaterialien sowie Schwermetall und/oder Schadstoff kontaminierte Böden, bereitzustellen, welches einerseits erheblich schneller und damit auch in der Regel kostengünstiger als im Stand der Technik erfolgen kann, andererseits aber auch besonders verlässliche und aussagekräftige Informationen zu der Menge, Art, Bindecharakteristik und der Rate der Freisetzung der in der Probe enthaltenen Schwermetalle und/oder Schadstoffe bereitstellt, zum Beispiel insbesondere über die weitere Prozessierung oder Weiterbehandlungsmöglichkeiten des untersuchten Materials Aufschluss gibt und somit eine Prognose zur Schadstoff- oder Schwermetallfreisetzung aus der Probe erlaubt.

Die vorliegende Erfindung löst das ihr zugrunde liegende Problem durch die Bereitstellung eines Verfahrens gemäß Hauptanspruch. Die vorliegende Erfindung löst das ihr zugrunde liegende Problem insbesondere durch die Bereitstellung eines Verfahrens zur Bestimmung von Schwermetallionen und Schadstoffen in einer Probe, insbesondere einer Probe von Abfall- und Altlastenmaterialien sowie Schwermetall und/oder Schadstoff kontaminierten Böden mittels Elektro-Ultrafiltration und mittels einer Elektro-Ultrafiltrationsvorrichtung umfassend eine Probenkammer, die über mit jeweils einer Ultrafiltrationsmembran versehene Öffnungen mit jeweils mindestens einer angrenzenden anodischen Außenkammer und mindestens einer angrenzenden kationischen Außenkammer verbunden ist, wobei:
a) die Probe in der Probenkammer mit einem mindestens ein Komplexierungsmittel enthaltendem wässrigen Medium vermischt, insbesondere suspendiert, wird,
b) die so bearbeitete Probe einer Initial-Elektro-Ultrafiltration (Initial-EUF) bei einer Initialtemperatur, einer elektrischen Initialspannung und einer Initialstromstärke unterzogen,
c) mindestens jeweils ein Initialanoden- und Initialkathodenfiltrat erhalten,
d) die Probe anschließend einer, sich in zumindest einer der EUF-Bedingungen von der Initial-Elektro-Ultrafiltration unterscheidenden, Folge-Elektro-Ultrafiltration (Folge-EUF) bei einer Folgetemperatur, einer elektrischen Folgespannung und einer Folgestromstärke unterzogen,
e) mindestens jeweils ein Folgeanoden- und Folgekathodenfiltrat erhalten und
f) die in den erhaltenen Fraktionen enthaltenen Schwermetallionen und/oder Schadstoffe analytisch, insbesondere qualitativ und/oder quantitativ, bestimmt werden,
wobei die Verfahrensschritte b) und c) zwei- bis zehnmal, vorzugsweise drei- bis neunmal, vorzugsweise fünfmal hintereinander und anschließend die Verfahrensschritte d) und e) zwei- bis zehnmal, vorzugsweise drei- bis neunmal, vorzugsweise fünfmal hintereinan wiederholt werden, um eine entsprechende Anzahl von Initialfraktionen und Folgefraktionen zu erhalten und
wobei der Füllstand in der Probenkammer während der Elektro-Ultrafiltration konstant gehalten wird.

In einer besonders bevorzugten Ausführungsform ist vorgesehen, in Schritt f) bei jeder einzelnen der erhaltenen Fraktionen aus den Schritten c) und e) die enthaltenen Schwermetallionen und/oder Schadstoffe analytisch, insbesondere qualitativ und/oder quantitativ, zu bestimmen.

Die vorliegende Erfindung sieht demgemäß ein Verfahren vor, gemäß dem die Probe in ein mindestens ein Komplexierungsmittel aufweisendes wässriges Medium eingebracht und aus diesem mittels mindestens zwei, sich in mindestens einem Verfahrensparameter unterscheidenden Elektro-Ultrafiltrationen, nämlich mindestens einer Initial-EUF und mindestens einer Folge-EUF, Schwermetallionen und/oder Schadstoffe extrahiert werden. Durch die erfindungsgemäß vorgesehene Anwesenheit mindestens eines Komplexierungsmittels in dem wässrigen Medium wird die Löslichkeit der zu analysierenden Schwermetallionen und/oder Schadstoffe erheblich erhöht und dementsprechend Schwermetallionen- oder andere Komplexe, insbesondere wasserlösliche Schwermetallionen- oder andere Komplexe, gebildet.

Durch die erfindungsgemäß vorgesehene Variation der Elektro-Ultrafiltrationsbedingungen wird es erfindungsgemäß in besonders vorteilhafter Weise ermöglicht, die unterschiedliche Verfügbarkeit und damit auch das Belastungspotential der untersuchten Proben, insbesondere Abfall- und Altlastenmaterialien sowie Schwermetall und/oder Schadstoff kontaminierte Böden näher zu analysieren, beispielsweise im Rahmen einer Initial-EUF unter schonenden Bedingungen zunächst leichter lösliche Schwermetall- und/oder Schadstoffionen zu extrahieren, um dann in einer Folge-EUF unter beispielsweise harscheren Bedingungen fester in der Probe gebundene Schwermetall- und/oder Schadstoffionen zu extrahieren, die das längerfristige Gefährdungspotential einer Probe repräsentieren. Auf diese Art und Weise wird es ermöglicht, für in einer Probe enthaltene Schwermetall- und/oder Schadstoffionen die Kinetik der Freisetzung als Freisetzungsraten, mittels Desorptionskurven, in Abhängigkeit von Bedingungen und Zeiträumen zu erstellen, die Aussagen über eine zeitliche Dynamik der Freisetzung in der Probe, das heißt insbesondere aus dem Abfall- und Altlastenmaterial sowie Schwermetall und/oder Schadstoff kontaminierten Böden, erlauben.

Insbesondere ermöglicht das erfindungsgemäße Verfahren den Erhalt mindestens einer Freisetzungskinetik für Schwermetall- und/oder Schadstoffionen, die allein von der Zeit abhängig ist, da jeweils, also in der Initial-EUF und der Folge-EUF, mindestens zwei Messwerte bei konstanten EUF-Bedingungen erhalten werden. Im Zusammenhang mit der vorliegenden Erfindung wird unter einer Freisetzungskinetik vorzugsweise die Beschreibung der Freisetzung eines Schwermetalls oder Schadstoffs aus einem Material in Abhängigkeit von der Zeit, insbesondere allein von der Zeit, verstanden. Dies ermöglicht in vorteilhafter Weise eine Prognose über die zukünftige zeitliche Freisetzungsdynamik der bestimmten Schwermetall- und/oder Schadstoffionen. Aufgrund einer solchen Prognose kann dann über die weitere Prozessierung bzw. das Gefährdungspotential des untersuchten Materials besser entschieden werden. Die Erstellung einer Kinetik, bestehend aus jeweils mindestens zwei Messwerten ist besonders vorteilhaft, da Schwermetall- und/oder Schadstoffionen aus unterschiedlichen Materialien oder Matrizes unterschiedlich schnell freigesetzt werden.

Die Erfindung sieht also vorteilhafterweise vor, die Proben in einem mindestens ein gelöstes Komplexierungsmittel aufweisenden wässrigen Medium zumindest zwei unterschiedlichen Bedingungen der Elektro-Ultrafiltration auszusetzen, nämlich mindestens einer Initial-Elektro-Ultrafiltration und mindestens einer Folge-Elektro-Ultrafiltration. Im Zusammenhang mit der vorliegenden Erfindung wird unter einer Elektro-Ultrafiltration, die aufgrund eines an die Probe angelegten elektrischen Feldes ausgelöste und bevorzugt durch Temperatur und Unterdruck unterstützte Wanderung von Ionen durch eine Ultrafiltrationsmembran, im Folgenden auch Ultrafilter oder Ultramembran bezeichnet, verstanden. Die erfindungsgemäß vorgesehene Elektro-Ultrafiltration einer Probe führt aufgrund des eingesetzten elektrischen Feldes zur Extraktion von Schwermetallionen und/oder Schadstoffen aus der Probe durch eine unerwünschte Teilchen und Substanzen zurückhaltende Ultramembran hindurch in einen räumlich von der Probe abgetrennten Bereich.

Im Zusammenhang mit der vorliegenden Erfindung ist vorgesehen, zwei unterschiedliche Elektro-Ultrafiltrationsbedingungen einzustellen, nämlich einerseits zunächst eine Initial-Elektro-Ultrafiltration mit bestimmten Bedingungen und andererseits anschließend eine Folge-Elektro-Ultrafiltration mit zumindest einer davon abweichenden Bedingung durchzuführen. Die sich unterscheidenden Bedingungen sind in bevorzugter Ausführung vorzugsweise die eingesetzten Elektro-Ultrafiltrationstemperaturen, die eingesetzte Spannung oder Stromstärke, die Elektro-Ultrafiltrationsdauer oder eine Kombination davon. In besonders bevorzugter Art und Weise kann zum Beispiel vorgesehen sein, die Initial- und die Folge-Elektro-Ultrafiltrationszeit identisch zu wählen und für die Initial-Elektro-Ultrafiltration eine geringere Temperatur und eine geringere Spannung und Stromstärke als für die Folge-Elektro-Ultrafiltration einzusetzen.

Erfindungsgemäß ist es vorgesehen, die Initial-Elektro-Ultrafiltration mehrstufig durchzuführen, das heißt, die zu untersuchende Probe zwei- bis zehnmal, insbesondere drei- bis neunmal, vorzugsweise fünfmal hintereinander zur Gewinnung von einer entsprechenden Anzahl von Initialfraktionen zu Elektro-Ultrafiltrieren und die Folge-Elektro-Ultrafiltration in mehreren Stufen, zwei- bis zehnmal, vorzugsweise drei- bis neunmal, vorzugsweise fünfmal hintereinander durchzuführen, das heißt eine entsprechende Anzahl von Folgefraktionen zu erhalten. Jede einzelne dieser Stufen wird im Folgenden auch als Elektro-Ultrafiltrationsschritt oder Extraktionsschritt bezeichnet. In besonders bevorzugter Ausführungsform ist vorgesehen, mindestens zwei Initial-EUF und/oder mindestens zwei Folge-EUF durchzuführen, vorzugsweise jeweils bei konstanten Bedingungen. Auf diese Weise kann bei jeweils konstanten Bedingungen sowohl bei der Initial- als auch der Folge-EUF eine Freisetzungskinetik erstellt werden, da jeweils in der Initial- als auch der Folge-EUF allein der zeitliche Verlauf die Freisetzung beeinflusst.

Für jedes analysierte Schwermetall- oder Schadstoffion wird also jeweils eine Initial-Freisetzungskinetik und eine Folge-Freisetzungskinetik erhalten, wobei die Initial-Freisetzungskinetik die kurzfristige Freisetzungsdynamik und die Folge-Freisetzungskinetik die längerfristige Freisetzungsdynamik des Schwermetall- oder Schadstoffions aus dem untersuchten Material über die Zeit repräsentiert.

Die bei jedem Extraktionsschritt erhaltenen Fraktionen, also zwei bis zehn Initial-EUF-Fraktionen und zwei bis zehn Folge-EUF-Fraktionen, werden jeweils einzeln auf ihren qualitativen und/oder quantitativen Gehalt an Schwermetall- und/oder Schadstoffionen hin analysiert.

Erfindungsgemäß wird das erfindungsgemäße Verfahren in einer Elektro-Ultrafiltrationsanlage durchgeführt, die eine Probenkammer und zumindest zwei jeweils über eine Öffnung mit der Probenkammer verbundenen Außenkammern aufweist. Die Probenkammer selbst dient der Aufnahme der zu analysierenden Probe und weist vorzugsweise eine Heizvorrichtung, vorzugsweise eine Rührvorrichtung, vorzugsweise eine Temperatur- und vorzugsweise eine Pegelsonde sowie vorzugsweise einen Wasserzulauf auf. Die Probenkammer ist mit den mindestens zwei, vorzugsweise benachbart angeordneten, Außenkammern über jeweils mindestens eine Öffnung verbunden, wobei die jeweils mindestens eine Öffnung durch jeweils eine Ultrafiltrationsmembran abgedeckt ist. Die Außenkammern sind als mindestens eine anodische und mindestens eine kathodische Außenkammer ausgeführt. In der anodischen Außenkammer ist eine Anode ausgebildet, vorzugsweise als Gitterelektrode, zum Beispiel eine Platingitteranode. In der kathodischen Außenkammer befindet sich eine Kathode, vorzugsweise eine als Gitterelektrode ausgebildete Kathode, vorzugsweise eine Platingitterkathode. Beide Außenkammern sind vorzugsweise mit einem Vakuumanschluss versehen, sodass auf diese Art und Weise die Extraktion durch Anlegen eines Unterdrucks beschleunigt werden kann.

Die Außenkammern weisen darüber hinaus Sammelabschnitte beziehungsweise Sammelbehälter auf, in denen das oder die Anodenbeziehungsweise Kathodenfiltrat beziehungsweise -filtrate gesammelt, abgenommen und einer analytischen Bestimmung zugeführt werden kann beziehungsweise können. Die analytische Bestimmung, zum Beispiel Konzentrationsbestimmung, kann beispielsweise mit Massenspektrometern oder Spektrophotometern geschehen.

Die vorliegende Erfindung betrifft daher vorteilhafterweise ein Verfahren zur Bestimmung von Schwermetallionen und/oder Schadstoffe in einer Probe mittels einer Elektro-Ultrafiltrationsvorrichtung, umfassend eine Probenkammer, die über mit jeweils einer Ultrafiltrationsmembran versehene Öffnungen mit jeweils mindestens einer angrenzenden anodischen Außenkammer und mindestens einer angrenzenden kathodischen Außenkammer verbunden ist und wobei die die Schwermetallionen und/oder Schadstoffe enthaltende Probe in die Probenkammer eingebracht, dort mit einem mindestens ein Komplexierungsmittel enthaltendem Extraktionsmedium vermischt, die Probe in einer Initial-Elektro-Ultrafiltration bei einer Initialtemperatur und einer elektrischen Initialspannung und Initialstromstärke so extrahiert wird, dass die Schwermetallionen und/oder Schadstoffe ihrer Ladung beziehungsweise der Ladung des gebildeten Schwermetallionen und/oder Schadstoff-Komplexes entsprechend durch die Ultrafiltrationsmembran aus der Probenkammer in die ihrer Ladung entsprechende anodische oder kathodische Außenkammer migrieren und die erhaltenen Anoden- beziehungsweise Kathodenfiltrate gesammelt, abgeführt und analytisch bestimmt werden und wobei anschließend die in der Probenkammer befindliche Probe einer Folge-Elektro-Ultrafiltration bei einer Folgetemperatur und einer elektrischen Folgespannung und Folgestromstärke so extrahiert wird, dass noch in der Probe vorhandene Schwermetallionen und/oder Schadstoffe entsprechend ihrer Ladung beziehungsweise der Ladung des gebildeten Schwermetallionen und/oder Schadstoff-Komplexes durch die Ultrafiltrationsmembran in die ihrer Ladung entsprechende anodische oder kathodische Außenkammer migrieren und das in der anodischen und kathodischen Kammer anfallende Anoden- beziehungsweise Kathodenfiltrat gesammelt, abgeführt und analytisch bestimmt werden.

Erfindungsgemäß wird die Initial-Elektro-Ultrafiltration insgesamt zwei- bis zehnmal, vorzugsweise drei- bis neunmal, insbesondere fünfmal, unmittelbar hintereinander zum Erhalt einer entsprechenden Anzahl von Initial-Fraktionen, insbesondere einzelnen Initial-Fraktionen, durchgeführt, so dass eine entsprechende Anzahl von Initialfraktionen gesammelt und analytisch bestimmt wird.

Erfindungsgemäß wird die Folge-Elektro-Ultrafiltration insgesamt zwei- bis zehnmal, vorzugsweise drei- bis neunmal, besonders bevorzugt fünfmal unmittelbar hintereinander durchgeführt, sodass eine entsprechende Anzahl von Folgefraktionen, insbesondere einzelnen Folge-Fraktionen, gesammelt und analytisch bestimmt werden.

Erfindungsgemäß werden somit zwei bis zehn, insbesondere fünf, Initial-Elektro-Ultrafiltrationen unmittelbar hintereinander und anschließend zwei bis zehn, vorzugsweise fünf, Folge-Elektro-Ultrafiltrationen unmittelbar hintereinander durchgeführt, sodass eine Vielzahl an Fraktionen, zum Beispiel fünf Fraktionen aus einer Initial-Elektro-Ultrafiltration und fünf Fraktionen aus einer Folge-Elektro-Ultrafiltration für die analytische Bestimmung zur Verfügung stehen.

In einer besonders bevorzugten Ausführungsform ist vorgesehen, dass die Initialtemperatur 15 bis 40°C, vorzugsweise 20°C beträgt.

Die Erfindung betrifft in einer bevorzugten Ausführung auch ein vorgenanntes Verfahren, wobei die Folgetemperatur 45 bis 95°C, insbesondere 80°C beträgt.

In einer besonders bevorzugten Ausführungsform ist vorgesehen, dass das Verfahren mit einer Initialtemperatur von 15 bis 40°C, insbesondere 20°C, und einer Folgetemperatur von 45 bis 95°C, insbesondere 80°C durchgeführt wird.

In einer besonders bevorzugten Ausführungsform ist vorgesehen, in der Initial-Elektro-Ultrafiltration eine Spannung von 50 bis 400 V, vorzugsweise 100 bis 300 V, insbesondere von maximal 200 V, vorzugsweise 100 bis 200 V einzusetzen. In einer weiteren bevorzugten Ausführungsform ist vorgesehen, für die Folge-Elektro-Ultrafiltration eine Spannung von 100 bis 800 V, vorzugsweise 200 bis 600 V, insbesondere von maximal 400 V, vorzugsweise 200 bis 400 V einzusetzen.

In einer besonders bevorzugten Ausführungsform ist vorgesehen, bei einem Elektrodenabstand von 2 bis 8 cm, vorzugsweise 4 cm, in der Initial-Elektro-Ultrafiltration eine Spannung von 50 bis 400 V, vorzugsweise 100 bis 300 V, insbesondere von maximal 200 V, vorzugsweise 100 bis 200 V einzusetzen. In einer weiteren bevorzugten Ausführungsform ist vorgesehen, bei einem Elektrodenabstand von 2 bis 8 cm, vorzugsweise 4 cm, für die Folge-Elektro-Ultrafiltration eine Spannung von 100 bis 800 V, vorzugsweise 200 bis 600 V, insbesondere von maximal 400 V, vorzugsweise 200 bis 400 V einzusetzen.

In einer weiteren bevorzugten Ausführungsform ist vorgesehen, im Rahmen der Initial-Elektro-Ultrafiltration eine Stromstärkenbegrenzung von 5 bis 100 mA, vorzugsweise 20 bis 50 mA, insbesondere von maximal 15 mA vorzusehen. In einer weiteren bevorzugten Ausführungsform ist vorgesehen, im Rahmen der Folge-Elektro-Ultrafiltration eine Stromstärkenbegrenzung von 50 bis 500 mA, vorzugsweise 100 bis 300 mA, insbesondere maximal 150 mA vorzusehen.

In einer besonders bevorzugten Ausführungsform ist vorgesehen, die zu analysierende Probe mit dem das mindestens eine Komplexierungsmittel enthaltenden wässrigen Medium, insbesondere Wasser, in eine homogene Mischung zu überführen, insbesondere diese unter mechanischer Agitation, zum Beispiel Rühren zu vermischen, insbesondere zu suspendieren.

In bevorzugter Ausführung wird das Verfahren insbesondere die Initial- und die Folge-Elektro-Ultrafiltration bei Unterdruck durchgeführt.

Vorzugsweise wird das erfindungsgemäße Verfahren mit einem Unterdruck von -0,5 bis -0,8 bar, vorzugsweise -0,7 bar in der anodischen Außenkammer und -0,1 bis -0,4 bar, vorzugsweise -0,3 bar in der kathodischen Außenkammer durchgeführt.

In einer besonders bevorzugten Ausführungsform wird als Ultrafiltrationsmembran eine Membran aus Polysulfonfaser, Polyethersulfon, Polypropylen oder insbesondere eine Cellulosemembran aus Nitrozellulose, vorzugsweise aus Triacetat als Triacetatmembran beziehungsweise ein Triacetatfilter verwendet.

In einer besonders bevorzugten Ausführungsform weist die Ultrafiltrationsmembran eine Porengröße von kleiner oder gleich 100.000 Da, vorzugsweise kleiner oder gleich 30.000 Da, vorzugsweise kleiner oder gleich 20.000 Da auf.

In einer weiteren bevorzugten Ausführungsform ist vorgesehen, jeden einzelnen Extraktionsschritt innerhalb der Initial-Elektro-Ultrafiltration für einen Zeitraum von 2 bis 10, vorzugsweise 3 bis 8, insbesondere 5 Minuten durchzuführen. In einer weiteren bevorzugten Ausführungsform ist vorgesehen, jeden einzelnen Extraktionsschritt innerhalb der Folge-Elektro-Ultrafiltration für einen Zeitraum von 2 bis 10, vorzugsweise 3 bis 8, insbesondere 5 Minuten durchzuführen.

In einer weiteren bevorzugten Ausführungsform ist vorgesehen, als Komplexierungsmittel insbesondere einen Chelatbildner einzusetzen.

In einer weiteren bevorzugten Ausführungsform ist insbesondere vorgesehen, als Komplexierungsmittel EDTA (Ethylendiamintetraessigsäure) beziehungsweise DTPA (Diethylentriaminpentaessigsäure) oder deren Salze einzusetzen. Weitere im erfindungsgemäßen Verfahren bevorzugt einsetzbare Komplexierungsmittel sind:
Ethylendiamin-di-(5-carboxy-2-hydroxyphenyl)essigsäure (EDDCHA), Ethylendiamin-di(o-hydroxyphenyl)essigsäure (EDDHA), Ethylendiamin-di-(o-hydroxy-p-methylphenyl)essigsäure (EDDHMA), Nitrilotriessigsäure (NTA) Ethylendiamintetraessigsäure-Dinatriumsalz (EDTA-Na2), Cyclohexandiamintetraessigsäure (CDTA), Etheylenglykol- bis (aminoethylether)-N,N,N',N'-Tetraessigsäure (EGTA), N-(2-Hydroxylethyl)-ethylendiamin-N,N,N' N'-triessigsäure-Trinatriumsalz (HEDTA),
Triethylentetraminhexaessigsäure (TTHA)
Gluconsäure
Isoascorbinsäure
Natriumisoascorbat
Weinsäure
Zitronensäure und ihre Salze Natriumcitrat, Kaliumcitrat und Calciumcitrat
Iminodisuccinat Tetranatriumsalz
Triethanolamin

In besonders bevorzugter Ausführungsform ist das wässrige das Komplexierungsmittel enthaltende Medium Wasser, insbesondere Leitungswasser, vorzugsweise voll entsalztes Wasser, zum Beispiel Osmosewasser oder Aqua dest.

In besonders bevorzugter Ausführungsform ist das untersuchte Material Abfall- und Altlastenmaterial wie Bauschutt, Bleihüttengranulat, Gießereirestsand, Pochsand oder Müllverbrennungsasche sowie Schwermetall und/oder Schadstoff kontaminierte Böden oder Material von Straßenbanketten, z.B. Bankettschälgut.

In einer besonders bevorzugten Ausführungsform ist das Verhältnis Probe zu dem Komplexierungsmittel enthaltenden wässrigen Medium 1:2 bis 1:1000, vorzugsweise 1:2 bis 1:100, insbesondere 1:10 (bezogen auf Gewicht).

Erfindungsgemäß ist vorgesehen, dass der Füllstand in der Probenkammer während der erfindungsgemäßen Ultrafiltration zum Beispiel mittels eines Sensors und eines Zulaufs konstant gehalten wird, das heißt bei der Extraktion abgeführtes wässriges Medium ersetzt wird.

In einer besonders bevorzugten Ausführungsform sind die mit der vorliegenden Verfahrensweise bestimmten Schwermetallionen und/oder Schadstoffe enthaltend oder bestehend aus N, C, P, K, Ca, Mg, Na, S, B, Li7, Be9, Sc45, V51, Cr53, Mn55, Fe56, Co59, Ni60, Cu65, Zn66, Ge74, As75, Se77, Y89, Zr90, Nb93, Mo98, Ru101, Rh103, Cd114, Sn118, Sb121, Rb85, Sr88, In115, Cs133, Ba137, La139, Ce140, Pr141, Nd143, Sm147, Eu151, Cd157, Tb159, Dy161, Ho165, Er166, Tm169, Yb173, Lu175, Hf177, Hf179, Ta181, W182, W183, Re185, Hg201, Tl205, Pb208, Bi209, Th232 und/oder U238.

In einer bevorzugten Ausführungsform der Erfindung können insbesondere auch Schadstoffe bestimmt werden, vorzugsweise anorganische oder organische Schadstoffe.

Weitere vorteilhafte Ausführungsformen der vorliegenden Erfindung ergeben sich aus den Unteransprüchen.

Die Erfindung wird im Folgenden anhand von Beispielen und der dazugehörigen Figuren näher erläutert.

Die Figur 1 zeigt eine Vorrichtung zur Durchführung des erfindungsgemäßen Verfahrens.

Die Figur 2 zeigt das Ergebnis einer Pb-Bestimmung in verschiedenen Materialien gemäß der Erfindung.

### Beispiel 1 - Versuchsaufbau

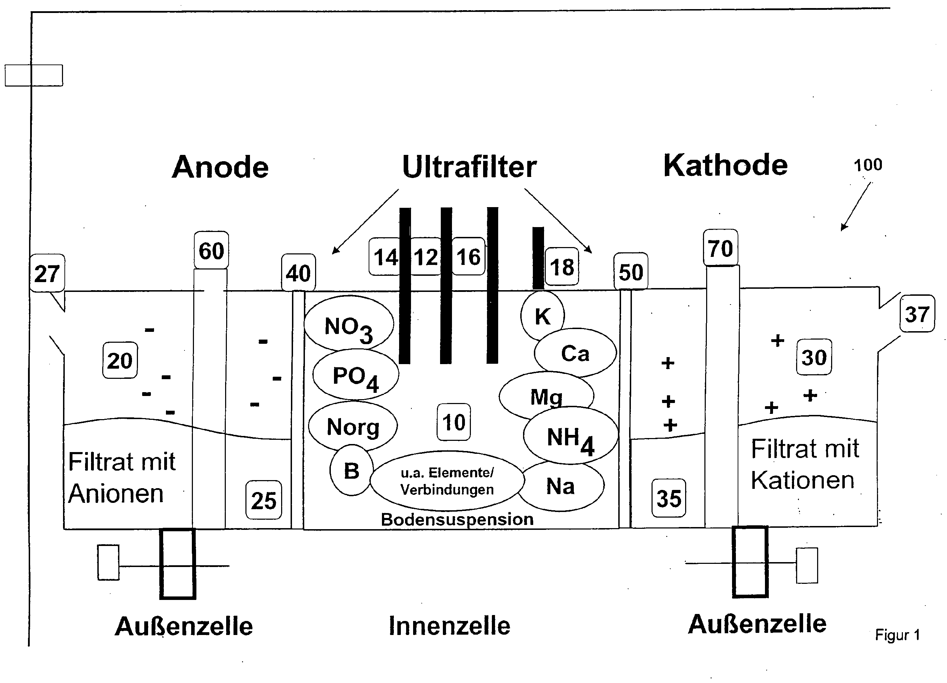

Die Figur 1 zeigt eine Elektro-Ultrafiltrationsvorrichtung 100, umfassend eine Probenkammer 10, eine anodische Außenkammer 20 und eine kathodische Außenkammer 30. Die Probenkammer 10 dient der Aufnahme einer hier nicht dargestellten, zu analysierenden Probe und weist eine Heizung 12, einen Rührer 14, eine Temperatursonde 16 und einen Wasserzulauf 18 auf. Die Probenkammer 10 ist von der anodischen Außenkammer 20 durch eine Ultrafiltrationsmembran 40 mit einer Porengröße von 20.000 Da in Form eines Triacetatfilters getrennt. Die Probenkammer 10 ist von der kathodischen Außenkammer 30 ebenfalls über eine gleichermaßen ausgebildete Ultrafiltrationsmembran 50 getrennt. In der anodischen Außenkammer 20 befindet sich eine als Platingitterelektrode 60 ausgebildete Anode. In der kathodischen Außenkammer 30 befindet sich eine als Platingitterelektrode 70 ausgebildete Kathode. Die anodische Außenkammer 20 weist darüber hinaus einen Sammelbehälter 25 für das Anodenfiltrat und die kathodische Außenkammer 30 einen Sammelbehälter 35 für das Kathodenfiltrat auf. Darüber hinaus weist die anodische Außenkammer 20 einen Vakuumanschluss 27 und die kathodische Außenkammer 30 einen Vakuumanschluss 37 auf.

Die erfindungsgemäße Verfahrensweise stellt sich wie folgt dar.

In die Probenkammer 10 wird eine 0,002 M DTPA wässrige Lösung eingebracht. Anschließend erfolgt das Einbringen einer zu analysierenden Probe, die mittels des Rührers 14 und nach Einstellen der notwendigen Temperatur mittels der Heizung 12 und der Temperatursonde 16 mit der Komplexierungslösung vermischt und suspendiert wird. Durch die Zugabe von DTPA werden in der Probe vorhandene Schwermetallionen und/oder Schadstoffe komplexiert und damit extrahierbar gemacht, insbesondere in Form eines stabilen geladenen Komplexes.

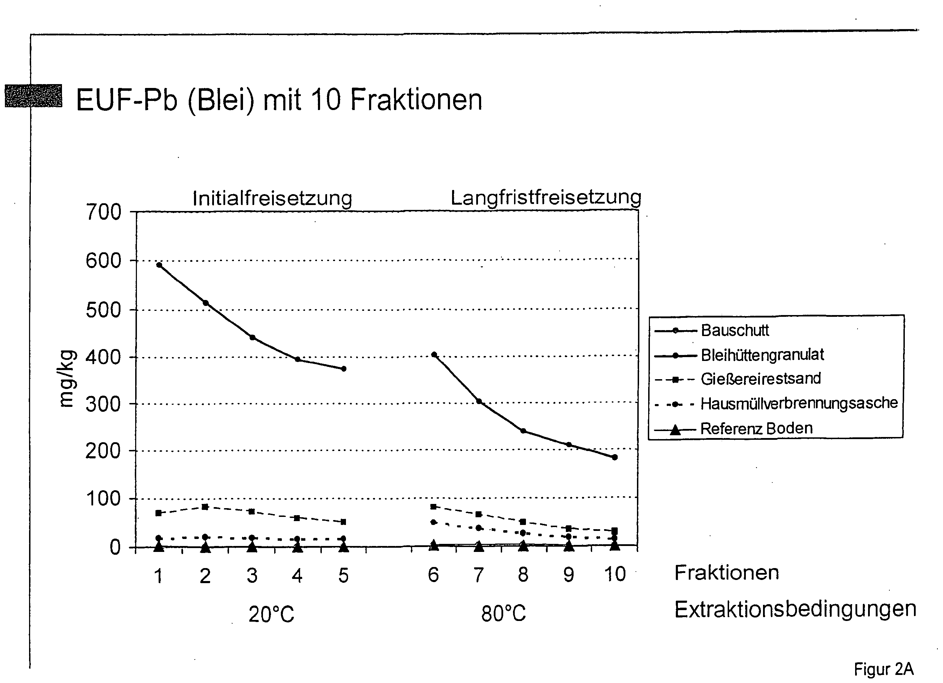

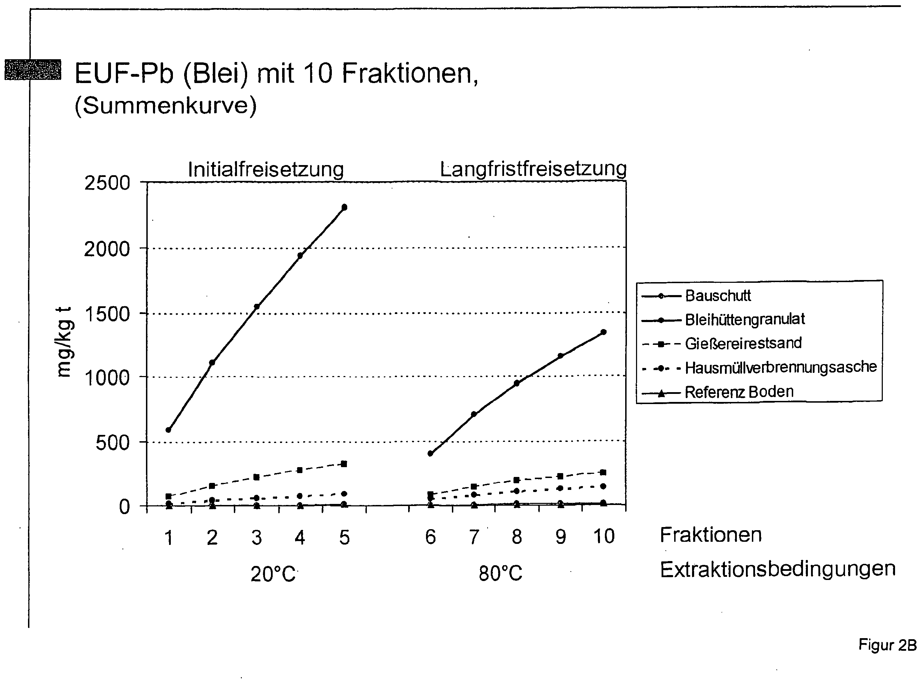

Bei Anlegen einer Spannung von 200 V für 5 Minuten mit einer Stromstärkenbegrenzung von maximal 15 mA und einer Temperatur von etwa 20°C wird ein elektrisches Feld für die Initial-EUF ausgebildet, das auf die in der Probenkammer befindliche Suspension einwirkt und dazu führt, dass sich die in der wässrigen Phase gelösten Kationen durch die Ultrafiltrationsmembran 50 hindurch in die kathodische Außenkammer 30 und die Anionen durch die Ultrafiltrationsmembran 40 in die anodische Außenkammer 20 bewegen. An den jeweiligen Elektroden werden die Ionen zusammen mit den jeweiligen elektrolytischen Spaltprodukten des Wassers in einem wässrigen Eluat aufgefangen und aus den Sammelbehältern 25 beziehungsweise 35 entnommen und bestimmt. Anschließend kann die Abfolge dieser Verfahrensschritte, zum Beispiel viermal, wiederholt werden, sodass jeweils fünf Anoden- und Kathodenfiltrate (Fraktionen 1-5 in Figur 2) erhalten werden. Über den Zulauf der (Komplexierungsmittel enthaltenden) wässrigen Lösung 18 und einem nicht dargestellten Pegelgeber wird durch externen Zulauf der Stand der wässrigen Lösung in der Probenkammer 10 konstant gehalten.

Anschließend werden unter geänderten Elektro-Ultrafiltrationsbedingungen mehrere Folge-Elektro-Ultrafiltrationsschritt(e) durchgeführt. Demgemäß wird in der Probenkammer eine Temperatur von etwa 80°C und eine Spannung von 400 V sowie eine maximale Stromkraftbegrenzung von 150 mA eingestellt und für jeweils 5 Minuten ein bis fünf Folge-EUF-Schritte zum Erhalt von ein bis fünf weiteren Filtraten (fünf Anoden- und Kathodenfiltrate in Figur 2, Fraktionen 6-10), die jeweils gesondert einer analytischen Bestimmung zugeführt werden können, durchgeführt.

Die Ergebnisse dieser Elektro-Ultrafiltration für die Materialien Bauschutt, Bleihüttengranulat, Gießereirestsand und Hausmüllverbrennungsasche für die Bestimmung von Pb sind in der Figur 2 dargestellt. Figur 2A zeigt die Pb-Mengen pro Filtrat (Fraktion), Figur 2B die aufsummierten freigesetzten Pb-Mengen.

## Patentansprüche

1. Verfahren zur Bestimmung von Schwermetallionen und/oder Schadstoffen in einer Probe mittels Elektro-Ultrafiltration und mittels einer Elektro-Ultrafiltrationsvorrichtung umfassend eine Probenkammer, die über mit jeweils einer Ultrafiltrationsmembran versehene Öffnungen mit jeweils mindestens einer angrenzenden anodischen Außenkammer und mindestens einer angrenzenden kationischen Außenkammer verbunden ist, wobei
a) die Probe in der Probenkammer mit einem mindestens ein Komplexierungsmittel enthaltenden wässrigen Medium vermischt wird,
b) die mit dem Medium vermischte Probe bei einer Initialtemperatur, einer Initialspannung und einer Initialstromstärke einer Initial-Elektro-Ultrafiltration unterzogen,
c) mindestens jeweils ein Initialanoden- und Initialkathodenfiltrat erhalten,
d) die mit dem Medium vermischte Probe anschließend bei einer Folgetemperatur, einer Folgespannung und einer Folgestromstärke einer, sich in zumindest einer der EUF-Bedingungen von der Initial-Elektro-Ultrafiltration unterscheidenden, Folge-Elektro-Ultrafiltration unterzogen,
e) mindestens jeweils ein Folgeanoden- und Folgekathodenfiltrat erhalten und
f) die in den erhaltenen Filtraten enthaltenen Schwermetallionen und/oder Schadstoffen analytisch bestimmt werden,
wobei die Verfahrensschritte b) und c) sowie die Verfahrensschritte d) und e) jeweils zwei- bis zehnmal unmittelbar hintereinander wiederholt werden, um eine entsprechende Anzahl von Initialfraktionen und Folgefraktionen zu erhalten und
wobei der Füllstand in der Probenkammer während der Elektro-Ultrafiltration konstant gehalten wird.

2. Verfahren nach Anspruch 1, wobei die Initial- Elektro-Ultrafiltration und die Folge-Elektro-Ultrafiltration jeweils bei konstanten Bedingungen durchgeführt werden.

3. Verfahren nach Anspruch 1 oder 2, wobei die Initialtemperatur 15 bis 40°C beträgt.

4. Verfahren nach einem der vorhergehenden Ansprüche, wobei die Folgetemperatur 45 bis 95°C beträgt.

5. Verfahren nach einem der vorhergehenden Ansprüche, wobei die Initialspannung bis 200 V beträgt.

6. Verfahren nach einem der vorhergehenden Ansprüche, wobei die Folgespannung bis 400 V beträgt.

7. Verfahren nach einem der vorhergehenden Ansprüche, wobei die Initial-Stromstärke bis 15 mA beträgt.

8. Verfahren nach einem der vorhergehenden Ansprüche, wobei die Folge-Stromstärke bis 150 mA beträgt.

9. Verfahren nach einem der vorhergehenden Ansprüche, wobei die die Schwermetallionen und/oder Schadstoff enthaltende Probe Abfall- und Altlastenmaterial sowie Schwermetall und Schadstoff kontaminierter Boden ist.

10. Verfahren nach einem der vorhergehenden Ansprüche, wobei die Elektro-Ultrafiltration durch eine Ultrafiltrationsmembran mit einem Porendurchmesser von 20.000 Da durchgeführt wird.

11. Verfahren nach einem der vorhergehenden Ansprüche, wobei die Initial- und die Folge-Elektro-Ultrafiltration bei Unterdruck durchgeführt wird.

## Claims

1. Method for determining heavy metal ions and/or pollutants in a sample by means of electro ultrafiltration and by means of an electro ultrafiltration device comprising a sample chamber that is connected by means of openings, which each are provided with an ultrafiltration membrane, to at least one adjacent anodic external chamber and at least one adjacent cationic external chamber each, whereby
a) the sample is mixed in the sample chamber with an aqueous medium that contains at least one complexing agent;
b) the sample mixed with the medium is subjected to an initial electro ultrafiltration at an initial temperature, an initial voltage, and an initial current;
c) at least one initial anode filtrate and initial cathode filtrate is obtained;
d) the sample mixed with the medium is subsequently subjected to a follow-up electro ultrafiltration at a follow-up temperature, a follow-up voltage, and a follow-up current, whereby the follow-up electro ultrafiltration differs from the initial electro ultrafiltration in at least one of the EUF conditions;
e) at least one follow-up anode filtrate and follow-up cathode filtrate is obtained;
f) the heavy metal ions and/or pollutants contained in the filtrates thus obtained are determined analytically,
whereby procedural steps b) and c) as well as procedural steps d) and e) each are repeated two to ten times in direct succession in order to obtain a corresponding number of initial fractions and follow-up fractions, and
whereby the filling level in the sample chamber is kept constant during the electro ultrafiltration.

2. Method according to claim 1, whereby the initial electro ultrafiltration and the follow-up electro ultrafiltration each are carried out at constant conditions.

3. Method according to claim 1 or 2, whereby the initial temperature is 15 to 40 °C.

4. Method according to any one of the preceding claims, whereby the follow-up temperature is 45 to 95 °C.

5. Method according to any one of the preceding claims, whereby the initial voltage is up to 200 V.

6. Method according to any one of the preceding claims, whereby the follow-up voltage is up to 400 V.

7. Method according to any one of the preceding claims, whereby the initial current is up to 15 mA.

8. Method according to any one of the preceding claims, whereby the follow-up current is up to 150 mA.

9. Method according to any one of the preceding claims, whereby the sample containing the heavy metal ions and/or pollutant is waste and contaminated site material as well as soil contaminated with heavy metal and pollutant.

10. Method according to any one of the preceding claims, whereby the electro ultrafiltration is carried out through an ultrafiltration membrane with a pore diameter of 20,000 Da.

11. Method according to any one of the preceding claims, whereby the initial and the follow-up electro ultrafiltration are carried out at a negative pressure.

## Revendications

1. Procédé de détermination d'ions métalliques lourds et/ou de matières polluantes dans un échantillon au moyen d'électro-ultrafiltration et au moyen d'un dispositif d'électro-ultrafiltration comprenant une chambre d'échantillons qui est connectée par le biais d'ouvertures pourvues chacune d'une membrane d'ultrafiltration à chaque fois à au moins une chambre externe anodique adjacente et au moins une chambre externe cationique adjacente, dans lequel
a) l'échantillon dans la chambre d'échantillons est mélangé à un milieu aqueux contenant au moins un agent de complexation,
b) l'échantillon mélangé au milieu est soumis à une électro-ultrafiltration initiale à une température initiale, une tension électrique initiale et une intensité électrique initiale,
c) au moins un filtrat d'anode initial et de cathode initial est à chaque fois obtenu,
d) l'échantillon mélangé au milieu est ensuite soumis à une électro-ultrafiltration consécutive se différenciant de l'électro-ultrafiltration initiale dans au moins une des conditions de EUF à une température consécutive, une tension électrique consécutive et une intensité électrique consécutive,
e) au moins un filtrat d'anode consécutif et de cathode consécutif est à chaque fois obtenu, et
f) les ions métalliques lourds et/ou matières polluantes contenus dans les filtrats obtenus sont déterminés de manière analytique,
dans lequel les étapes de procédé b) et c) ainsi que les étapes de procédé d) et e) sont à chaque fois répétées deux à dix fois directement les unes derrière les autres pour obtenir un nombre correspondant de fractions initiales et de fractions consécutives, et
dans lequel le niveau dans la chambre d'échantillons est maintenu constant pendant l' électro-ultrafiltration.

2. Procédé selon la revendication 1, dans lequel l'électro-ultrafiltration initiale et l'électro-ultrafiltration consécutive sont chacune réalisées à des conditions constantes.

3. Procédé selon la revendication 1 ou 2, dans lequel la température initiale va de 15 à 40 °C.

4. Procédé selon une des revendications précédentes, dans lequel la température consécutive va de 45 à 95 °C.

5. Procédé selon une des revendications précédentes, dans lequel la tension électrique initiale va jusqu'à 200 V.

6. Procédé selon une des revendications précédentes, dans lequel la tension électrique consécutive va jusqu'à 400 V.

7. Procédé selon une des revendications précédentes, dans lequel l'intensité électrique initiale va jusqu'à 15 mA.

8. Procédé selon une des revendications précédentes, dans lequel l'intensité électrique consécutive va jusqu'à 150 mA.

9. Procédé selon une des revendications précédentes, dans lequel l'échantillon contenant les ions métalliques lourds et/ou la matière polluante est du matériau de déchets et de sites pollués ainsi que du sol contaminé par du métal lourd et de la matière polluante.

10. Procédé selon une des revendications précédentes, dans lequel l'électro-ultrafiltration est réalisée par une membrane d'ultrafiltration avec un diamètre de pores de 20 000 Da.

11. Procédé selon une des revendications précédentes, dans lequel l'électro-ultrafiltration initiale et consécutive est réalisée avec une pression négative.
